# EUROPEAN PATENT SPECIFICATION

(11) **EP 3 227 261 B1**
(45) Date of publication and mention of the grant of the patent: **03.07.2019**
(21) Application number: 14812157.7
(22) Date of filing: 02.12.2014
(51) Int. Cl.: C07C 69/76, C08K 5/12

(54) **ESTER PLASTICIZERS BASED ON ETHYLHEXANOL AND PROPYLHEPTANOL**
ESTER-WEICHMACHER AUF DER BASIS VON ETHYLHEXANOL UND PROPYLHEPTANOL
PLASTIFIANTS À BASE D'ESTER CONTENANT DE L'ÉTHYLHEXANOL ET DU PROPYLHEPTANOL

(43) Date of publication of application: 11.10.2017
(73) Proprietor: Emery Oleochemicals GmbH, 40599 Düsseldorf (DE)
(72) Inventor: DAUTE, Peter, 27616 Beverstedt (DE); SCHAEFER, Martin, 27616 Stubben (DE)
(74) Representative: Kinkeldey, Daniela
(86) International application number: PCT/EP2014/076288
(87) International publication number: WO 2016/086977

(56) References cited:
- JP-A- H05 214 159
- US-A1- 2014 096 703

## Description

The present invention relates to a triester obtainable by reacting (a) an alcohol mixture consisting of 20-50 wt% ethylhexanol, 40-64 wt% propylheptanol and 0-20 wt% of other alcohols with (b) an aromatic tricarboxylic acid. Further aspects of the invention relate to a thermoplastic polymer comprising the triester according to the invention and uses of said triester.

### BACKGROUND OF THE INVENTION

Plasticizers are additives that increase the flexibility, pliability, and plasticity or fluidity of a material. The main applications are for plastics, especially thermoplastic compositions. Plasticizers are useful for a variety of polymers and applications such as wire-and-cable insulations, wire-and-cable jackets, coatings, adhesives, and castings - just to name some applications.

In order to change the mechanical properties of a material plasticizers can be admixed. Thereby, flexible and plastic compounds can be obtained. For example, to the thermoplastic polymer polyvinyl chloride (PVC) a plasticizer can be added in such a large amount that PVC can be processed as a paste (see Gächter/Müller, Kunstoffadditive, 2. Auflage Carl Hanser Verlag).

For applications that need to resist higher temperatures plasticizers such as phthalates and aromatic tricarboxylic esters including trimellitic acid esters can be used. Known in the prior art is for example the compound tri-2-ethylhexyl trimellitate. One disadvantage of this compound, however, is that it is still volatile. Thus, the utility of this plasticizer is reduced in particular for applications that need to resist higher temperatures, since at such temperatures the plasticizer will evaporate from the thermoplastic polymer. However, the permanence over time of a plasticizer in the polymer, even when exposed to relatively high temperatures is an important requirement for many technical applications involving plastic compositions.

With respect to phthalates prior art plasticizers include for example di(ethyl hexyl)phthalate ("DEHP"), di-isononyl phthalate ("DINP"), and other phthalate plasticizers. However, phthalate plasticizers are in general less frequently used since this plasticizer type is linked to concerns over potential adverse health effects.

Plasticizers are particularly useful if they have a reduced volatility at higher temperatures but have at the same time also a low pour point. A low pour point ensures that the final product comprising the plasticizer remains flexible even at low temperatures. A reduced volatility at higher temperatures will also preserve the plasticizer's effect in the product also at high temperatures as is required for example for thermoplastic polymer compositions used for the electrical insulation of automotive electrical components. Thus, a plasticizer having the aforementioned properties will increase the versatility of its use.

US 2014/0096703 discloses plasticizer, plasticizer composition, heat-resistant resin composition and method for preparing the same.

JP H05214159 discloses mixed alcohols for plasticizer and plasticizer produced therefrom.

Thus, there is a general need in the art for novel plasticizers that do not have the above outlined disadvantages.

### SUMMARY OF THE INVENTION

The present invention provides in a first aspect a triester obtainable by reacting
(a) an alcohol mixture consisting of
   (i) 20-50 wt% ethylhexanol, based on the total weight of the alcohol mixture
   (ii) 40-64 wt% propylheptanol, based on the total weight of the alcohol mixture and
   (iii) 0-20 wt% other alcohols, based on the total weight of the alcohol mixture
   with
(b) an aromatic tricarboxylic acid or anhydride thereof.

Preferably said ethylhexanol is 2- ethylhexanol and said other alcohols is a decanol that is different from 2-propylheptanol or an alcohol mixture comprising such decanols.

Also provided is a thermoplastic polymer comprising the triester according to the invention.

A further aspect of the invention relates to the use of the triester of the invention as plasticizer in a thermoplastic polymer.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Some documents are cited throughout the text of this specification. Nothing herein is to be construed as an admission that the invention is not entitled to antedate such disclosure by virtue of prior invention.

Novel plasticizers were identified that have a reduced volatility at higher temperatures and are also characterized by a low pour point. Accordingly, the invention provides in a first aspect a triester obtainable by reacting
(a) an alcohol mixture consisting of
   (i) 20-50 wt% ethylhexanol, based on the total weight of the alcohol mixture
   (ii) 40-64 wt% propylheptanol, based on the total weight of the alcohol mixture and
   (iii) 0-20 wt% other alcohols, based on the total weight of the alcohol mixture
   with
(b) an aromatic tricarboxylic acid or anhydride thereof.

The components (i), (ii) and (iii) together are in total 100 wt% of the alcohol mixture, i.e. the alcohol mixture (a) of the invention consists of components (i), (ii) and (iii). In a preferred embodiment of the ester, said aromatic tricarboxylic acid is selected from the group consisting of trimellitic acid, hemimellitic acid, trimesic acid, naphthalenetricarboxylic acid, anthracenetricarboxylic acid, biphenyltricarboxylic acid, benzophenonetricarboxylic acid and mixtures and position isomers of the aforementioned acids.

According to the present invention the alcohol mixture consists of
(i) 20-50 wt% ethylhexanol, based on the total weight of the alcohol mixture; and
(ii) 40-64 wt% propylheptanol, based on the total weight of the alcohol mixture; and
(iii) 0-20 wt% other alcohols, based on the total weight of the alcohol mixture.

"Other alcohols" as used herein are preferably alcohols other than those used as components (i) and (ii). In a more preferred embodiment, "other alcohols" are branched primary alcohols and even more preferably a decanol other than 2-propylheptanol or an alcohol mixture comprising such decanols.

Most preferably, "other alcohols" refers to a branched primary decanol different from 2-propylheptanol or refers to an alcohol mixture consisting of two or more of such branched primary decanols.

In a further preferred embodiment of the ester according to the invention the alcohol mixture consists of 30-45 wt% ethylhexanol, based on the total weight of the alcohol mixture; and the remaining alcohols in the alcohol mixture consist of propylheptanol and other alcohols, wherein said other alcohols are not more than maximally 20 wt% of the total weight of the alcohol mixture.

It is further preferred that the alcohol mixture consists of 30-45 wt% ethylhexanol, based on the total weight of the alcohol mixture; and the remaining alcohols in the alcohol mixture consist of propylheptanol and other alcohols, wherein said other alcohols are not more than maximally 15 wt% of the total weight of the alcohol mixture.

In a more preferred embodiment the alcohol mixture consists of 30-35 wt% ethylhexanol, based on the total weight of the alcohol mixture; and the remaining alcohols in the alcohol mixture consist of propylheptanol and other alcohols, wherein said other alcohols are not more than maximally 15 wt% of the total weight of the alcohol mixture.

In a further preferred embodiment, the alcohol mixture consists of 38-50 wt% ethylhexanol, based on the total weight of the alcohol mixture; and the remaining alcohols in the alcohol mixture consist of propylheptanol and other alcohols, wherein said other alcohols are not more than maximally 15 wt% of the total weight of the alcohol mixture.

In a more preferred embodiment the alcohol mixture consists of 40-45 wt% ethylhexanol, based on the total weight of the alcohol mixture; and the remaining alcohols in the alcohol mixture consist of propylheptanol and other alcohols, wherein said other alcohols are not more than maximally 12 wt% of the total weight of the alcohol mixture.

Alternatively, the triester of the invention may also be based on an alcohol mixture that consists of 20-37 wt% ethylhexanol, based on the total weight of the alcohol mixture; and the remaining alcohols in the alcohol mixture consist of propylheptanol and other alcohols, wherein said other alcohols are not more than maximally 16 wt% of the total weight of the alcohol mixture.

In a particularly preferred embodiment of the ester according to the invention said ethylhexanol is 2- ethylhexanol and said propylheptanol is 2- propylheptanol.

A further aspect of the invention relates to a thermoplastic polymer comprising the triester according to the invention.

Thereby it is preferred that the thermoplastic polymer is selected from the group consisting of polyethylene, PET, PC, ABS, polyamide, TPU, TPE, nitrile butadiene rubber (NBR), hydrogenated nitrile butadiene rubber (HNBR), chloroprene rubber (CR), polystyrene and a polyolefin, preferably a halogenated polyolefin such as polyvinyl chloride.

Preferably, the thermoplastic polymer is a halogenated polymer. More preferably, the halogenated polymer is a PVC polymer selected from the group consisting of PVC homopolymers, PVC copolymers, polyvinyl dichlorides (PVDC), and polymers of vinylchloride with vinyl, acrylic and other co-monomers.

In a further aspect the invention provides the use of the triester of the invention as defined herein as plasticizer in a thermoplastic polymer.

A preferred embodiment of the use according to the invention relates to a use, wherein the thermoplastic polymer is selected from the group consisting of polyethylene, PET, PC, ABS, polyamide, TPU, TPE, nitrile butadiene rubber (NBR), hydrogenated nitrile butadiene rubber (HNBR), chloroprene rubber (CR), polystyrene and a polyolefin, preferably a halogenated polyolefin such as polyvinyl chloride.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments.

The following examples are merely illustrative of the present invention and should not be construed to limit the scope of the invention as indicated by the appended claims in any way.

### EXAMPLES

### Example 1: Ester Production

The amount of 2-ethylhexanol ("2 EH") and 2-propylheptanol ("2 PH") indicated in table 1 below was heated under nitrogen with trimellitic acid anhydride ("TMA"; respective amounts see table) and 500 ppm of tin-(II)-oxalate (Evonic), based on the total amount of the composition. The 2-propylheptanol that was used was of technical grade purity and therefore consisted of 80% 2-propylheptanol and 20% decanol isomers other than 2-propylheptanol. The esterification reaction begins at about 150°C with the formation of water and the temperature is slowly increased to 220°C. After 4 hours the reaction water was further removed and the vacuum was reduced over 10 hours to about 5 mbar. The reaction was stopped when the acid number dropped below 1. Remaining unreacted alcohol was removed by distillation. Thereafter, the product was filtered.

**Table 1: Amounts of compounds used**

| Example | 2 EH in g | 2 PH in g | TMA in g | Yield in g |
|---|---|---|---|---|
| 1 | 996 | 0 | 352 | 998 |
| 2 | 682 | 415 | 403 | 1163 |
| 3 | 990 | 1370 | 837 | 2430 |
| 4 | 343 | 755 | 406 | 1228 |
| 5 | 0 | 1134 | 367 | 1153 |

### Example 2: Determination of Physical Properties of the Ester

The degree of discoloration of the ester was measured in Hazen units according to the method described in DIN EN ISO 6271-2005.

The acid number was determined according to DIN 53402.

The viscosity was measured using DIN 53019.

Furthermore, the pourpoint, which indicates the lowest temperature at which a liquid becomes semi solid and loses its flow characteristics, was determined according to DIN ISO 3016(10/82).

The cloud point was determined according to DIN EU 23015.

Volatility of the ester was tested using a Netzsch instrument for thermogravimetry by heating a sample under N₂ from 20°C to a final temperature of 300°C at a rate of at a 10 K/min. After the sample reached a temperature of 300°C the sample was kept at this temperature for 30 minutes.

The results are shown in table 2 and 3 below:

**Table 2:**

| Example | Colour Hazen | Acid number (mg KOH/g) | Calculation index 20°C | Viscosity 20°C (mPa·s) |
|---|---|---|---|---|
| 1 | 95 | 0,2 | 1,4853 | 300 |
| 2 | 96 | 0,09 | 1,4844 | 333 |
| 3 | 88 | 0,12 | 1,4837 | 364 |
| 4 | 108 | 0,11 | 1,4835 | 368 |
| 5 | 159 | 0,74 | 1,4826 | 401 |

**Table 3**

| Example | pour-point | % weight loss of ester after heat treatment as described | cloud point |
|---|---|---|---|
| 1 | -42 | 93% | -25 |
| 2 | -36 | n.d. | n.d. |
| 3 | -40 | 70% | -35 |
| 4 | -45 | n.d. | n.d. |
| 5 | -35 | 56% | -17 |

Example 3 is according to the invention.

### Example 3: Thermoplastic Composition Comprising Ester

The following compositions were prepared:

**Table 4: Preparation of test specimens**

| Example: | B6 | B7 | B8 | B9 | B10 |
|---|---|---|---|---|---|
| Polyvinyl chloride | 100 | 100 | 100 | 100 | 100 |
| Edenol D 81 (epoxidized soybean oil) | 3 | 3 | 3 | 3 | 3 |
| Stabiol CZ 2222 (Ca/Zn-based stabilizer) | 2 | 2 | 2 | 2 | 2 |
| Ester of example 1 | 40 | - | - | - | - |
| Ester of example 2 | - | 40 | - | - | - |
| Ester of example 3 | - | - | 40 | - | - |
| Ester of example 4 | - | - | - | 40 | - |
| Ester of example 5 | - | - | - | - | 40 |

The components as shown above were mixed together and milled on a laboratory rolling mill for 5 minutes at 185°C. Using rolled sheets 3mm press plates were produced in a laboratory press at 180°C and on these test specimens were examined according to DIN ISO 868 for their Shore A hardness (three measurements per sample). The results are outlined in table 5 below:

| Example: | B6 | B7 | B8 | B9 | B10 |
|---|---|---|---|---|---|
| Shore A | 94,8 | 96,9 | 96,9 | 97,6 | 97,8 |
| | 96,3 | 96,6 | 96,9 | 98,1 | 97,7 |
| | 95,1 | 96,6 | 96,9 | 98,0 | 97,7 |

### Example B8 is according to the invention.

As can be seen from the examples above, the optimized ester of the invention can be used as plasticizer in the processing of thermoplastic resins. The ester shows good compatibility with the polymer, low volatilities and also provides excellent low temperature properties.

## Claims

1. Triester obtainable by reacting
(a) an alcohol mixture consisting of
(i) 20-50 wt% ethylhexanol, based on the total weight of the alcohol mixture;
(ii) 40-64 wt% propylheptanol, based on the total weight of the alcohol mixture; and
(iii) 0-20 wt% other alcohols, based on the total weight of the alcohol mixture;
with
(b) an aromatic tricarboxylic acid or anhydride thereof.

2. Triester according to claim 1, wherein the aromatic tricarboxylic acid is selected from the group consisting of trimellitic acid, hemimellitic acid, trimesic acid, naphthalenetricarboxylic acid, anthracenetricarboxylic acid, biphenyltricarboxylic acid, benzophenonetricarboxylic acid and mixtures and position isomers of the aforementioned acids.

3. Triester according to any of the preceding claims, wherein the alcohol mixture consists of
30-45 wt% ethylhexanol, based on the total weight of the alcohol mixture; and
the remaining alcohols in the alcohol mixture consist of propylheptanol and other alcohols, wherein said other alcohols are not more than maximally 20 wt% of the total weight of the alcohol mixture.

4. Triester according to any of the preceding claims, wherein the alcohol mixture consists of
30-45 wt% ethylhexanol, based on the total weight of the alcohol mixture; and
the remaining alcohols in the alcohol mixture consist of propylheptanol and other alcohols, wherein said other alcohols are not more than maximally 15 wt% of the total weight of the alcohol mixture.

5. Trister according to any of the preceding claims, wherein the alcohol mixture consists of
38-50 wt% ethylhexanol, based on the total weight of the alcohol mixture; and
the remaining alcohols in the alcohol mixture consist of propylheptanol and other alcohols, wherein said other alcohols are not more than maximally 15 wt% of the total weight of the alcohol mixture.

6. Triester according to any of the preceding claims, wherein the alcohol mixture consists of
20-37 wt% ethylhexanol, based on the total weight of the alcohol mixture; and
the remaining alcohols in the alcohol mixture consist of propylheptanol and other alcohols, wherein said other alcohols are not more than maximally 16 wt% of the total weight of the alcohol mixture.

7. Triester according to any of the preceding claims, wherein ethylhexanol is 2-ethylhexanol and propylheptanol is 2-propylheptanol.

8. Thermoplastic polymer comprising the triester according to any of claims 1-7.

9. Thermoplastic polymer according to claim 8, wherein the thermoplastic polymer is selected from the group consisting of polyethylene, PET, PC, ABS, polyamide, TPU, TPE, nitrile butadiene rubber (NBR), hydrogenated nitrile butadiene rubber (HNBR), chloroprene rubber (CR), polystyrene and a polyolefin, preferably a halogenated polyolefin such as polyvinyl chloride.

10. Use of the triester of any of claims 1-7 as plasticizer in a thermoplastic polymer.

11. Use according to claim 10, wherein the thermoplastic polymer is selected from the group consisting of polyethylene, PET, PC, ABS, polyamide, TPU, TPE, nitrile butadiene rubber (NBR), hydrogenated nitrile butadiene rubber (HNBR), chloroprene rubber (CR), polystyrene and a polyolefin, preferably a halogenated polyolefin such as polyvinyl chloride.

## Patentansprüche

1. Triester, der erhältlich ist durch Umsetzen von
(a) einer Alkoholmischung, bestehend aus
(i) 20-50 Gew.-% Ethylhexanol, bezogen auf das Gesamtgewicht der Alkoholmischung,
(ii) 40-64 Gew.-% Propylheptanol, bezogen auf das Gesamtgewicht der Alkoholmischung,
(iii) 0-20 Gew.-% anderen Alkoholen, bezogen auf das Gesamtgewicht der Alkoholmischung,
mit
(b) einer aromatischen Tricarbonsäure oder einem Anhydrid davon.

2. Triester nach Anspruch 1, wobei die aromatische Tricarbonsäure aus der Gruppe bestehend aus Trimellitsäure, Hemimellitsäure, Trimesinsäure, Naphthalintricarbonsäure, Anthracentricarbonsäure, Biphenyltricarbonsäure, Benzophenontricarbonsäure und Mischungen und Stellungsisomeren der oben genannten Säuren ausgewählt ist.

3. Triester nach einem der vorhergehenden Ansprüche, wobei die Alkoholmischung aus
30-45 Gew.-% Ethylhexanol, bezogen auf das Gesamtgewicht der Alkoholmischung, und
die übrigen Alkohole in der Alkoholmischung aus Propylheptanol und anderen Alkoholen bestehen, wobei die anderen Alkohole nicht mehr als maximal 20 Gew.-% des Gesamtgewichts der Alkoholmischung ausmachen, besteht.

4. Triester nach einem der vorhergehenden Ansprüche, wobei die Alkoholmischung aus
30-45 Gew.-% Ethylhexanol, bezogen auf das Gesamtgewicht der Alkoholmischung, und
die übrigen Alkohole in der Alkoholmischung aus Propylheptanol und anderen Alkoholen bestehen, wobei die anderen Alkohole nicht mehr als maximal 15 Gew.-% des Gesamtgewichts der Alkoholmischung ausmachen, besteht.

5. Triester nach einem der vorhergehenden Ansprüche, wobei die Alkoholmischung aus
38-50 Gew.-% Ethylhexanol, bezogen auf das Gesamtgewicht der Alkoholmischung, und
die übrigen Alkohole in der Alkoholmischung aus Propylheptanol und anderen Alkoholen bestehen, wobei die anderen Alkohole nicht mehr als maximal 15 Gew.-% des Gesamtgewichts der Alkoholmischung ausmachen, besteht.

6. Triester nach einem der vorhergehenden Ansprüche, wobei die Alkoholmischung aus
20-37 Gew.-% Ethylhexanol, bezogen auf das Gesamtgewicht der Alkoholmischung, und
die übrigen Alkohole in der Alkoholmischung aus Propylheptanol und anderen Alkoholen bestehen, wobei die anderen Alkohole nicht mehr als maximal 16 Gew.-% des Gesamtgewichts der Alkoholmischung ausmachen, besteht.

7. Triester nach einem der vorhergehenden Ansprüche, wobei es sich bei dem Ethylhexanol um 2-Ethylhexanol handelt und es sich bei dem Propylheptanol um 2-Propylheptanol handelt.

8. Thermoplastisches Polymer, umfassend den Triester nach einem der Ansprüche 1-7.

9. Thermoplastisches Polymer nach Anspruch 8, wobei das thermoplastische Polymer aus der Gruppe bestehend aus Polyethylen, PET, PC, ABS, Polyamid, TPU, TPE, Nitril-Butadien-Kautschuk (NBR), hydriertem Nitril-Butadien-Kautschuk (HNBR), Chloropren-Kautschuk (CR), Polystyrol und einem Polyolefin, vorzugsweise einem halogenierten Polyolefin wie Polyvinylchlorid, ausgewählt ist.

10. Verwendung des Triesters nach einem der Ansprüche 1-7 als Weichmacher in einem thermoplastischen Polymer.

11. Verwendung nach Anspruch 10, wobei das thermoplastische Polymer aus der Gruppe bestehend aus Polyethylen, PET, PC, ABS, Polyamid, TPU, TPE, Nitril-Butadien-Kautschuk (NBR), hydriertem Nitril-Butadien-Kautschuk (HNBR), Chloropren-Kautschuk (CR), Polystyrol und einem Polyolefin, vorzugsweise einem halogenierten Polyolefin wie Polyvinylchlorid, ausgewählt ist.

## Revendications

1. Triester qui peut être obtenu en faisant réagir
(a) un mélange d'alcools qui se compose de
(i) 20-50 % en poids d'éthylhexanol, relativement au poids total du mélange d'alcools ;
(ii) 40-64 % en poids de propylheptanol, relativement au poids total du mélange d'alcools ; et
(iii) 0-20 % en poids d'autres alcools, relativement au poids total du mélange d'alcools ;
avec
(b) un acide tricarboxylique aromatique ou un anhydride de celui-ci.

2. Triester selon la revendication 1, dans lequel l'acide tricarboxylique aromatique est sélectionné dans le groupe constitué de l'acide trimellitique, de l'acide hémimellitique, de l'acide trimésique, de l'acide naphtalènetricarboxylique, de l'acide anthracènetricarboxylique, de l'acide biphényltricarboxylique, de l'acide benzophénonetricarboxylique et de mélanges et d'isomères de position des acides susmentionnés.

3. Triester selon l'une quelconque des revendications précédentes, dans lequel le mélange d'alcools se compose de
30-45 % en poids d'éthylhexanol, relativement au poids total du mélange d'alcools ; et
les autres alcools dans le mélange d'alcools se composent de propylheptanol et d'autres alcools, lesdits autres alcools ne représentant pas plus de 20 % en poids au maximum du poids total du mélange d'alcools.

4. Triester selon l'une quelconque des revendications précédentes, dans lequel le mélange d'alcools se compose de
30-45 % en poids d'éthylhexanol, relativement au poids total du mélange d'alcools ; et
les autres alcools dans le mélange d'alcools se composent de propylheptanol et d'autres alcools, lesdits autres alcools ne représentant pas plus de 15 % en poids au maximum du poids total du mélange d'alcools.

5. Triester selon l'une quelconque des revendications précédentes, dans lequel le mélange d'alcools se compose de
38-50 % en poids d'éthylhexanol, relativement au poids total du mélange d'alcools ; et
les autres alcools dans le mélange d'alcools se composent de propylheptanol et d'autres alcools, lesdits autres alcools ne représentant pas plus de 15 % en poids au maximum du poids total du mélange d'alcools.

6. Triester selon l'une quelconque des revendications précédentes, dans lequel le mélange d'alcools se compose de
20-37 % en poids d'éthylhexanol, relativement au poids total du mélange d'alcools ; et
les autres alcools dans le mélange d'alcools se composent de propylheptanol et d'autres alcools, lesdits autres alcools ne représentant pas plus de 16 % en poids au maximum du poids total du mélange d'alcools.

7. Triester selon l'une quelconque des revendications précédentes, dans lequel l'éthylhexanol est le 2-éthylhexanol et le propylheptanol est le 2-propylheptanol.

8. Polymère thermoplastique comprenant le triester selon l'une quelconque des revendications 1 à 7.

9. Polymère thermoplastique selon la revendication 8, le polymère thermoplastique étant sélectionné dans le groupe constitué du polyéthylène, du PET, du PC, d'un composé ABS, du polyamide, d'un TPU, d'un TPE, du caoutchouc nitrile butadiène (NBR), du caoutchouc nitrile butadiène hydrogéné (HNBR), du caoutchouc chloroprène (CR), du polystyrène et d'une polyoléfine, préférablement une polyoléfine halogénée telle que le polychlorure de vinyle.

10. Utilisation du triester selon l'une quelconque des revendications 1 à 7 comme plastifiant dans un polymère thermoplastique.

11. Utilisation selon la revendication 10, dans laquelle le polymère thermoplastique est sélectionné dans le groupe constitué du polyéthylène, du PET, du PC, d'un composé ABS, du polyamide, d'un TPU, d'un TPE, du caoutchouc nitrile butadiène (NBR), du caoutchouc nitrile butadiène hydrogéné (HNBR), du caoutchouc chloroprène (CR), du polystyrène et d'une polyoléfine, préférablement une polyoléfine halogénée telle que le polychlorure de vinyle.
